# EUROPEAN PATENT SPECIFICATION

(11) **EP 2 918 306 B1**
(45) Date of publication and mention of the grant of the patent: **04.11.2020**
(21) Application number: 15156272.5
(22) Date of filing: 24.02.2015
(51) Int. Cl.: A61M 25/09

(54) **Guide wire**
Führungsdraht
Fil-guide

(30) Priority: 24.02.2014 JP 2014032993; 10.11.2014 JP 2014228130
(43) Date of publication of application: 16.09.2015
(73) Proprietor: ASAHI INTECC CO., LTD., Seto-shi, Aichi 489-0071 (JP)
(72) Inventor: Kanazawa, Yuya, Seto-shi, Aichi 489-0071 (JP)
(74) Representative: Winter, Brandl, Fürniss, Hübner, Röss, Kaiser, Polte - Partnerschaft mbB

(56) References cited:
- EP-A1- 2 005 988
- EP-A2- 2 505 225
- US-A- 3 452 742
- US-A- 4 854 330

## Description

### BACKGROUND OF THE INVENTION

### 1. Field of the Invention

The present invention relates to a guide wire used as a medical device inserted in a body cavity for treatment or examination.

### 2. Description of the Related Art

Conventionally, there are proposed various medical devices inserted in a tubular organ such as a blood vessel, a gastrointestinal tract, and a urinary duct, or a body tissue for treatment or examination.

For example, Japanese Unexamined Patent Application Publication No. 2007-501648 discloses a guide wire including a core wire and a coil body wound around an distal portion of the core wire. The coil body includes a straight section in a straight state from the proximal end toward the distal direction and a curved section arranged at a distal side of the straight section. EP2505225 A2 describes a guidewire with a core and a coil. The dital end portion of the guidewire is elastically deformed into a curved portion.

EP2005988 A1 describes a medical guide wire having a front helical spring which is formed by two stranded wire units in a helically-wired spring configuration in which the two stranded wire units are further twisted in a helically-wired multiple spring configuration.

### SUMMARY OF THE INVENTION

When a degree of a stenosis of a blood vessel is relatively serious as a stenosis portion typically known as a chronic total occlusion (CTO), it is preferable to shape a guide wire in considerably short range from the distal end in order to allow the guide wire to pass through a slight gap in the stenosis portion, as performed in the guide wire described in Japanese Unexamined Patent Application Publication No. 2007-501648.

However, in the guide wire including a curved section by such shaping, a fragment of a calcified lesion in a stenosis portion, for example, enters between a gap of element wires positioned in the curved section and causes interference, and the distal portion of the guide wire is suppressed to move and becomes difficult to move, that is, the situation may cause a stuck of the guide wire. Such the situation decreases the passing performance when the guide wire passes through the stenosis portion.

Even the guide wire described in Japanese Unexamined Patent Application Publication No. 2007-501648 may not sufficiently secure the passing performance when the guide wire passes through the stenosis portion due to the interference between the curved section and a lesion, and there is room for an improvement in this point.

In view of the above aspects, the present invention aims at providing a guide wire capable of avoiding a situation in which a distal portion of the guide wire is got caught and stuck in a stenosis portion and securing a sufficient passing performance in the stenosis portion.

In order to solve the above problem, the guide wire according to aspects of the present invention has been defined in the appended claims.

A guide wire according to the first aspect which does not form part of the invention is a guide wire including a core shaft and a coil body comprising a plurality of turns wound around a distal portion of the core shaft, wherein the coil body is formed by winding at least one winding element into a helical coil structure, the coil body includes a straight section in a straight state from a proximal end of the coil body toward a distal direction and a curved section arranged at a distal side of the straight section, and adjacent turns of the coil body are in contact with each other on an outer side of the curved section.

The second aspect of the present invention is the guide wire according to the first aspect, wherein each of the at least one winding element is formed of a single wire.

The third aspect of the present invention is the guide wire according to the first aspect, wherein each of the at least one winding element is obtained by stranding a plurality of wires.

The fourth aspect of the present invention is the guide wire according to any one of the first to third aspects, wherein the coil body is formed by winding a single winding element into a helical coil structure.

The fifth aspect of the present invention is the guide wire according to the first aspect, wherein the coil body comprises a first coil portion forming the curved section and a second coil portion forming the straight section, wherein the first coil portion is formed by winding a plurality of winding elements into a helical coil structure with each winding element being obtained by stranding a plurality of wires, and wherein the second coil portion is formed of a single wire winding element wound into a helical coil structure.

The sixth aspect of the present invention is the guide wire according to the fifth aspect, wherein a proximal end of the first coil portion and a distal end of the second coil portion are joined to each other.

The seventh aspect of the present invention is the guide wire according to any one of the first to third aspects, wherein the coil body is formed by winding a plurality of winding elements into a helical coil structure.

The eighth aspect of the present invention is the guide wire according to the seventh aspect, wherein a winding angle of first winding elements forming the curved section with respect to a longitudinal axis of the straight section is larger than a winding angle of second winding elements forming the straight section with respect to the longitudinal axis of the straight section.

The ninth aspect of the present invention is the guide wire according to the seventh aspect or the eighth aspect, wherein the first winding elements forming the curved section are partially arranged substantially in parallel.

The tenth aspect of the present invention is the guide wire according to any one of the seventh aspect to the ninth aspect, wherein the first winding elements forming the curved section are in pressure contact with each other.

The eleventh aspect of the present invention is the guide wire according to any one of the seventh aspect to the tenth aspect, wherein the straight section is tapered to a smaller diameter toward the curved section.

In the coil body used in the guide wire of the first aspect, the coil body comprises a plurality of turns (loops) formed by winding at least one winding element (a single winding element or a plurality of winding elements) into a helical coil structure so that adjacent turns of the coil body on the outer side of the curved section are in contact with each other. If the adjacent turns on the outer side of the curved section are separate from each other with a space, a fragment of a calcified lesion in a stenosis portion, for example, may enter into the gaps between adjacent turns of the at least one winding element and cause interference, and the distal portion of the guide wire may be suppressed to move, and the passing performance when the guide wire passes through the stenosis portion may decrease. However, in the first aspect, adjacent turns of the coil body on the outer side of the curved section are in contact with each other, which prevents a fragment of a lesion from entering between the adjacent turns in the curved section. As a result, the distal portion of the guide wire is hardly got caught, which sufficiently secures the passing performance when the guide wire passes through the stenosis portion.

In the guide wire of the second and third aspects, each of the at least one winding element is formed either of a single wire or is obtained by stranding a plurality of wires (the plurality of stranded wires may also be referred to as a "stranded wire") . In any case, the turns of the at least one winding element on the outside of the curved section are in contact with each other. If the turns on the outer side of the curved section are separate from each another and provided with a space, a fragment of a calcified lesion in a stenosis portion, for example, may enter between the element wires and cause interference, and the distal portion of the guide wire may be suppressed to move, and the passing performance when the guide wire passes through the stenosis portion may decrease. However, as the turns on the outer side of the curved section are in contact with each other, a fragment of a lesion is prevented from entering between the turns of the at least one winding element in the curved section. As a result, the distal portion of the guide wire is hardly got caught, which sufficiently secures the passing performance when the guide wire passes through the stenosis portion.

In the guide wire of the fourth aspect, the coil body is formed by winding a single winding element into a helical coil structure. The single winding element may be formed of a single wire or may be obtained by stranding a plurality of wires.

In the guide wire of the fifth aspect, the coil body is composed of a first coil portion and a second coil portion wherein the first coil portion is formed of a stranded winding element wound into a helical coil structure and the second coil portion is formed of a single wire winding element wound into a helical coil structure.

In the guide wire of the sixth aspect, the first coil portion and second coil portion are joined to each other, e.g. by applying a bonding material, in particular a brazing metal, to a border portion between a proximal end of the first coil portion and a distal end of the second coil portion.

In the guide wire of the seventh aspect, the coil body is formed by winding a plurality of winding elements into a helical coil structure. Each of the plurality of winding elements may formed of a single wire or may be obtained by stranding a plurality of wires (the plurality of stranded wires may also be referred to as a "stranded wire").

For example, in the guide wire of the eighth aspect, the coil body is formed by winding a plurality of winding elements, which are obtained by stranding single wires, into a helical coil structure. The stranded wires are in close contact with each other firmly, and thus even when the curved section is formed, the stranded wires do not separate from each other, and a gap is hardly generated. Therefore, it is possible to securely prevent a fragment of a lesion from entering the curved section.

Moreover, when the stress is added to the distal portion (the curved section) of the guide wire, relative slight movement is possible not only between stranded wires but also between element wires forming the stranded wires. Thus, the distal portion has a high degree of freedom and excellent flexibility while being resistant to plastic deformation and securing a preferable restoring characteristic. Consequently, excellent following property and selectivity are obtained even in a blood vessel bent three-dimensionally in a complicated manner. Furthermore, even when the distal portion of the guide wire hits a lesion having high hardness, the distal portion is not bent by stress exerted by the hit and the guide wire can be used continuously.

In the guide wire of the eighth aspect, a (stranding angle or) winding angle of the first winding elements, e.g. stranded wires, forming the curved section with respect to a longitudinal axis of the straight section is larger than a (stranding or) winding angle of the second winding elements, e.g. stranded wires, forming the straight section. In this manner, when the distal portion of the guide wire is curved, the first winding elements forming the curved section erect along a vertical direction, and come to press the second winding elements forming the straight section in an axial direction. That is, it is possible to secure the contact between the winding elements even in a part where a gap is easily generated between the winding elements (a boundary part between the straight section and the curved section) when the distal portion of the guide wire is curved.

The guide wire of the ninth aspect includes a part where the first winding elements, e.g. stranded wires, forming the curved section are arranged substantially in parallel. That is, such a structure prevents the first winding elements forming the curved section from deviating relatively from the adjacent first stranded wire and separating therefrom, and no gap is generated between the turns of the winding elements forming the curved section. This prevents a fragment of a lesion, for example, from entering between the turns of the winding elements in the curved section. Thus, the distal portion of the guide wire is hardly got caught, which secures the favorable passing performance when the guide wire passes through a stenosis portion.

In the guide wire of the tenth aspect, the first winding elements, e.g. stranded wires, forming the curved section are in pressure contact with each other. In this manner, it is possible to securely prevent generation of a gap between the turns of the winding elements forming the curved section of the guide wire.

In the eleventh aspect, the straight section of the coil body is tapered to a smaller diameter toward the curved section (the distal side). In this manner, the winding angle of the second winding elements, e.g. the stranding angle of the second stranded wires, forming the straight section becomes smaller toward the curved section, and the second winding elements come to press the first winding elements, e.g. stranded wires, forming the curved section. That is, the turns of the winding elements are in pressure contact with each other firmly at the border portion between the curved section and the straight section. As a result, it is possible to prevent more securely generation of a gap between the turns of the winding elements positioned at the border portion between the curved section and the straight section.

Moreover, the diameter is smallest at the proximal end part of the curved section, which improves the flexibility of the curved section. Therefore, even in a blood vessel bent three-dimensionally in a complicated manner, it is possible to sufficiently secure the blood vessel following property and the blood vessel selectivity of the distal portion of the guide wire.

In addition, in the straight section of the guide wire of the eleventh aspect, the diameter becomes larger from the distal side to the proximal side. Thus, when the press-in operation is performed, sliding resistance to a blood vessel wall, for example, becomes larger toward the proximal side. Therefore, if the distal portion (the curved section) of the guide wire penetrates through a blood vessel wall and projects outside the blood vessel, and the operation is still continued thereafter, the sliding resistance of the straight section to the blood vessel wall (a wall of the through-hole) becomes larger gradually, thus conveying such touch feeling to a hand of an operator.

Consequently, it becomes easy to stop the press-in operation in the guide wire once and introduce again the distal portion of the guide wire to a proper blood vessel. That is, the guide wire of the eleventh aspect allows insertion of the distal portion to a target region safely and accurately.

### BRIEF DESCRIPTION OF THE DRAWINGS

FIG. 1 is a partial cross-sectional enlarged view illustrating a first example not forming part of the invention of a guide wire of the present invention;
FIG. 2 is a partial cross-sectional enlarged view illustrating a second embodiment of the guide wire of the present invention;
FIG. 3 is a cross-sectional view taken from A-A of a coil body in FIG. 2;
FIG. 4 is a partial cross-sectional enlarged view illustrating a third embodiment of the guide wire of the present invention;
FIG. 5 is a partial enlarged view illustrating a curved section of a coil body in FIG. 4 and the vicinity thereof;
FIG. 6 is a partial cross-sectional enlarged view illustrating a fourth embodiment of the guide wire of the present invention; and
FIG. 7 is a partial enlarged view illustrating a curved section of a coil body in FIG. 6 and the vicinity thereof.

### DETAILED DESCRIPTION OF THE PREFERRED EMBODIMENTS

The guide wire of the present invention will be described based on preferred embodiments illustrated in the enclosed drawings.

### [First embodiment]

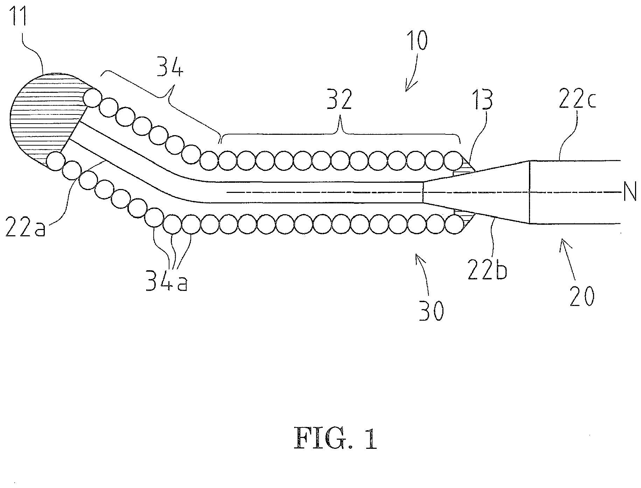

FIG. 1 is a partial cross-sectional enlarged view illustrating a first example of a guide wire not forming part of the present invention. In FIG. 1, the left side corresponds to a distal side to be inserted in a body, while the right side corresponds to a proximal side to be operated by a technician such as a physician. Note that FIG. 1 schematically illustrates the guide wire, and the proportion in FIG. 1 is different from actual one.

A guide wire 10 illustrated in FIG. 1 is used to facilitate positioning of a catheter in a cardiovascular system, for example. The guide wire 10 includes a core shaft 20 and a coil body 30 covering the outer periphery of the distal portion of the core shaft 20.

First, the core shaft 20 is described. The core shaft 20 includes a small diameter portion 22a, a taper portion 22b, and a large diameter portion 22c, in this order from the distal end to the proximal side. The small diameter portion 22a is a portion on the most distal end of the core shaft 20, and is a softest portion in the core shaft 20. The small diameter portion 22a is formed to be a flat plate shape by press working. The taper portion 22b is tapered with a circular cross-section, and is reduced in diameter toward the distal side. The large diameter portion 22c has a larger diameter than the small diameter portion 22a.

The material composing the core shaft 20 is not especially limited, and includes stainless steel (SUS304), a super-elastic alloy such as a Ni-Ti alloy, a piano wire, or a cobalt alloy, for example.

Next, the coil body 30 is described. The coil body 30 of the first embodiment is a single wound coil formed by winding a single wire into a helical coil structure. The coil body 30 comprises a plurality of turns (loops) 34a arranged one after the other in a longitudinal direction of the coil body 30. The single wire forming the coil body 30 is an example of a (single) winding element in the claims.

As illustrated in FIG. 1, the distal end of the coil body 30 is fixed to the distal end of the core shaft 20 by a distal side joint portion 11. The proximal end of the coil body 30 is fixed to the core shaft 20 by a proximal side joint portion 13. The material composing the distal side joint portion 11 and the proximal side joint portion 13 is not especially limited, and includes a brazing metal such as a Sn-Pb alloy, a Pb-Ag alloy, a Sn-Ag alloy, or an Au-Sn alloy, for example.

The coil body 30 includes a straight section 32 in a straight state from a proximal end toward the distal direction and a curved section 34 arranged at the distal side of the straight section 32. The curved section 34 is curved with a predetermined angle relative to an axial direction N of the straight section 32.

In the first example, adjacent turns 34a of the coil body 30 are in contact with each other on an outer side of the curved section 34. That is, on the outer side of the curved section 34, no gap is generated between adjacent turns 34a of the single wire.

Note that the outer side of the curved section 34 is a side opposite to the side to which the curved section 34 is curved, relative to the axial direction N. This applies also in the embodiments described later.

Here, if the turns 34a are separate from each another with a space or gap being provided between adjacent turns 34a on the outer side of the curved section 34, a fragment of a calcified lesion in a stenosis portion, for example, may enter between adjacent turns 34a and cause interference, and the distal portion of the guide wire 10 may be suppressed to move, and the passing performance when the guide wire passes through the stenosis portion may decrease. However, in the first example, the adjacent turns 34a on the outer side of the curved section 34 are in contact with each other, which prevents a part of a lesion from entering between the turns 34a in the curved section 34. As a result, the distal portion of the guide wire 10 is hardly got caught, which sufficiently secures the passing performance when the guide wire passes through the stenosis portion.

The material composing the coil body 30 is not especially limited, and the coil body 30 may be formed by a radiopaque wire or a radiotransparent wire. The material of the radiopaque wire is not especially limited, and includes gold, platinum, tungsten, or an alloy including such elements (e.g., a platinum-nickel alloy), for example. The material of the radiotransparent wire is not especially limited, and includes stainless steel (SUS304, SUS316, etc.), a super-elastic alloy such as a Ni-Ti alloy, or a piano wire, for example.

### [Second embodiment]

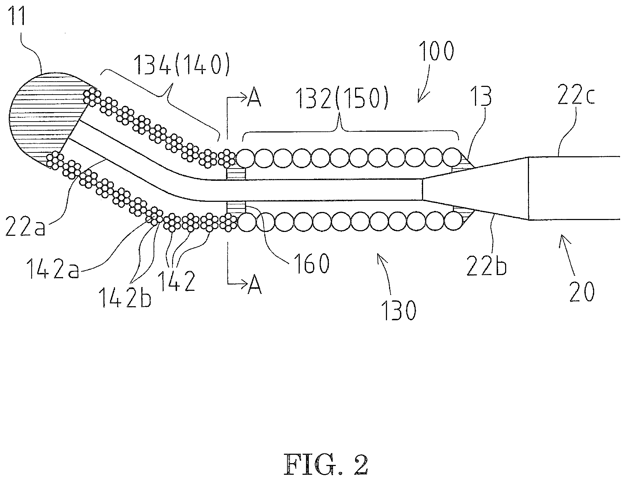

Next, the second embodiment of the guide wire of the present invention will be described with reference to FIG. 2. In FIG. 2, the left side corresponds to a distal side to be inserted in a body, while the right side corresponds to a proximal side to be operated by a technician such as a physician. Note that FIG. 2 schematically illustrates the guide wire, and the proportion in FIG. 2, including the configuration of the cross-section of the stranded wire, is different from actual one.

The first embodiment described above adopts a single wound coil formed by winding a single wire (single winding element) into a helical coil structure. By contrast, a guide wire 100 of the second embodiment includes a coil body 130 comprising a first coil portion 140 forming a curved section 134 and a second coil portion 150 forming a straight section 132. The first coil portion 140 forming the curved section 134 is formed by winding a plurality of stranded wires 142, which are each obtained by stranding a plurality of single wires 142a, 142b, into a helical coil structure. The plurality of stranded wires 142 is an example of a plurality of winding elements in the claims . Further, a single stranded wire 142 is an example of a single winding element in the claims.

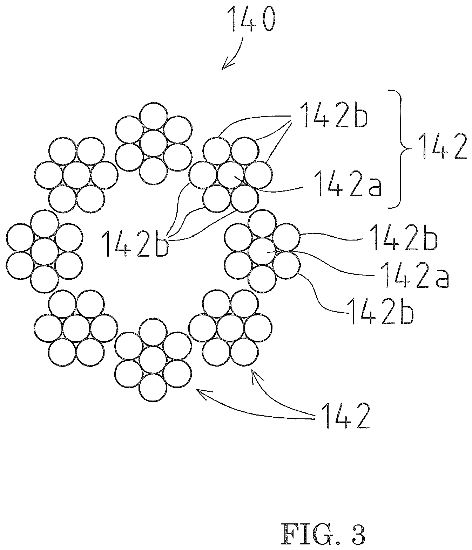

To be more specific, as illustrated in FIGS. 2 and 3, the first coil portion 140 forming the curved section 134 is formed by winding a plurality of (eight in the embodiment) the stranded wires 142, each of which includes a core wire 142a and six side wires 142b wound to cover the periphery of the core wire 142a, into a helical coil structure.

The material composing the core wire 142a and the side wires 142b is not especially limited, and includes stainless steel such as martensitic stainless steel, ferritic stainless steel, austenitic stainless steel, austenite-ferrite duplex stainless steel, or a precipitation hardening type stainless steel; a super-elastic alloy such as a Ni-Ti alloy; or platinum, gold, tungsten, tantalum, iridium, or an alloy including such elements, which are radiopaque metal impermeable to radiation such as X-rays, for example.

As described above, in the guide wire 100 of the second embodiment, the first coil portion 140 forming the curved section 134 is formed by winding the stranded wires 142, which are each obtained by stranding the single wires 142a, 142b, into a helical coil structure. In the curved section 134 having such a structure, the stranded wires 142 are in close contact with each other firmly. Thus, when the curved section 134 is formed, the stranded wires 142 do not separate from each other and a gap is hardly generated therebetween.

This prevents a fragment of a lesion from entering between adjacent turns in the curved section 134. As a result, the distal portion of the guide wire 100 is hardly got caught by the lesion, which sufficiently secures the passing performance when the guide wire passes through the stenosis portion.

When the stress is added to the distal portion (the curved section 134) of the guide wire 100, relative slight movement is possible not only between the stranded wires 142 but also between the single wires 142a, 142b forming the stranded wires 142. Thus, the distal portion has a high degree of freedom and excellent flexibility while being resistant to plastic deformation and securing a preferable restoring characteristic. Consequently, excellent following property and selectivity are obtained even in a blood vessel bent three-dimensionally in a complicated manner. Furthermore, even when the distal portion of the guide wire 100 hits a lesion having high hardness, the distal portion is not bent by stress exerted by the hit and the guide wire can be used continuously.

Meanwhile, the second coil body 150 forming the straight section 132 is formed by a single-wound coil obtained by winding a single wire into a helical coil structure. Such single wire is an example of a single winding element in the claims. The material composing the second coil body 150 is not especially limited, and the second coil body may be formed by a radiopaque wire or a radiotransparent wire. The material of the radiopaque wire is not especially limited, and includes gold, platinum, tungsten, or an alloy including such elements (e.g., a platinum-nickel alloy), for example. The material of the radiotransparent wire is not especially limited, and includes stainless steel (SUS304, SUS316, etc.), a super-elastic alloy such as a Ni-Ti alloy, and a piano wire, for example.

The proximal end of the first coil portion 140 and the distal end of the second coil portion 150 are joined to each other by an intermediate joint portion 160. The material composing the intermediate joint portion 160 is not especially limited, and includes a brazing metal such as a Sn-Pb alloy, a Pb-Ag alloy, a Sn-Ag alloy, and an Au-Sn alloy, for example.

### [Third embodiment]

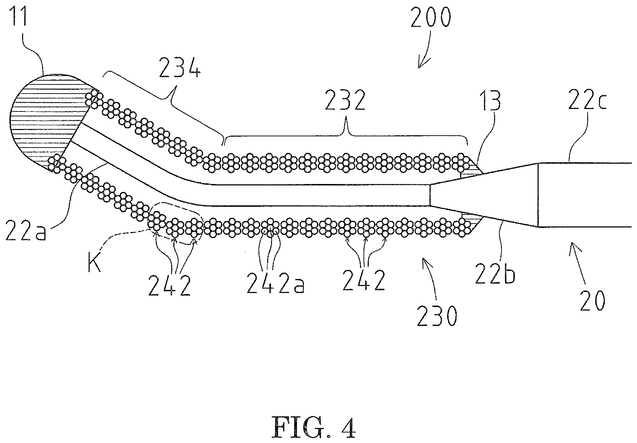

Next, the third embodiment of the guide wire of the present invention will be described with reference to FIG. 4. In FIG. 4, the left side corresponds to a distal side to be inserted in a body, while the right side corresponds to a proximal side to be operated by a technician such as a physician. Note that FIG. 4 schematically illustrates the guide wire, and the proportion in FIG. 4, including the configuration of the cross-section of the stranded wire, is different from actual one.

In the second embodiment described above, only the curved section of the coil body is formed by winding a plurality of stranded wires (winding elements), which are obtained by stranding a plurality of single wires, into a helical coil structure. By contrast, in a guide wire 200 of the third embodiment, not only a curved section 234 but also a straight section 232 is formed by winding a plurality of stranded wires 242, which are each obtained by stranding a plurality of single wires, into a helical coil structure. That is, in the third embodiment, the coil body 230 is configured entirely by winding a plurality of stranded wires 242, which are obtained by stranding a plurality of single wires 242a, into a helical coil structure. Note that the structure of the coil body 230 is same as the first coil portion of the second embodiment described above.

In this structure, the stranded wires 242 are in close contact with each other, similarly to the second embodiment, and a gap is hardly generated when the curved section 234 is formed. Particularly, the coil body 230 of the third embodiment is formed entirely by winding a plurality of the stranded wires 242, which are obtained by stranding a plurality of the single wires 242a, into a helical coil structure, and thus the stranded wires positioned at a border portion K between the straight section 232 and the curved section 234 are in pressure contact with each other firmly.

That is, even in a portion where a gap is generated easily when the curved section 234 is formed (the border portion K between the straight section 232 and the curved section 234), it is possible to effectively prevent generation of such the gap, and securely prevent a fragment of a lesion, for example, from entering between adjacent turns of the curved section 234. As a result, the distal portion of the guide wire 200 is hardly got caught by the lesion, which sufficiently secures the passing performance when the guide wire 200 passes through the stenosis portion.

Furthermore, in the proximal portion of the coil body 230 (the straight section 232), the single wires 242a are fastened by each other and the stranded wires 242 are also fastened by each other, which increases contact pressure and improves the adherence, when a rotational force is added. Consequently, the torque transmission performance is improved, and the preferable passing performance of the guide wire 200 is secured.

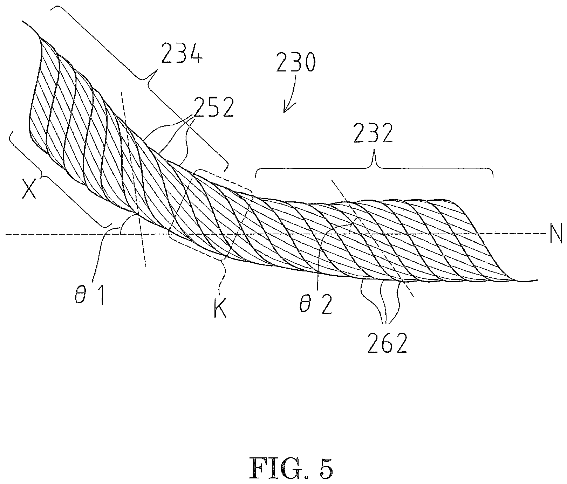

Here, the structure of the curved section 234 of the embodiment and the vicinity thereof will be described in detail with reference to FIG. 5.

As illustrated in FIG. 5, a stranding angle or winding angle θ1 of the first stranded wires 252 forming the curved section 234 is larger than a stranding angle or winding angle θ2 of the second stranded wires 262 forming the straight section 232. In this manner, when the distal portion of the guide wire 200 is curved, the first stranded wires 252 forming the curved section 234 erect along a vertical direction, and come to press the second stranded wires 262 forming the straight section 232 in an axial direction N.

Note that the stranding angle θ1 of the first stranded wires 252 is an angle on an acute angle side defined by the axial direction N of the straight section 232 and the winding direction of the first stranded wires 252, while the stranding angle θ2 of the second stranded wires 262 is an angle on an acute angle side defined by the axial direction N of the straight section 232 and the winding direction of the second stranded wires 262. This applies also in the embodiment described later.

That is, it becomes possible to secure contact between the stranded wires 252, 262 even in a portion where a gap is generated easily when the distal portion of the guide wire 200 is curved (the border portion K between the straight section 232 and the curved section 234).

Furthermore, the first stranded wires 252 forming the curved section 234 are arranged substantially in parallel in a part X. Such a structure prevents a first stranded wire 252 forming the curved section 234 from deviating relatively from an adjacent first stranded wire 252 and separating therefrom, and no gap is formed between turns of the stranded wires 252 forming the curved section 234.

This prevents a fragment of a lesion from entering between turns of the first stranded wires 252 forming the curved section 234. Thus, the distal portion of the guide wire 200 is hardly got caught, which secures the preferable passing performance when the guide wire 200 passes the stenosis portion.

Moreover, in the curved section 234, the adjacent first stranded wires 252 are in pressure contact with each other. In this manner, it is possible to securely prevent generation of a gap between the first stranded wires 252 forming the curved section 234 of the guide wire 200.

### [Fourth embodiment]

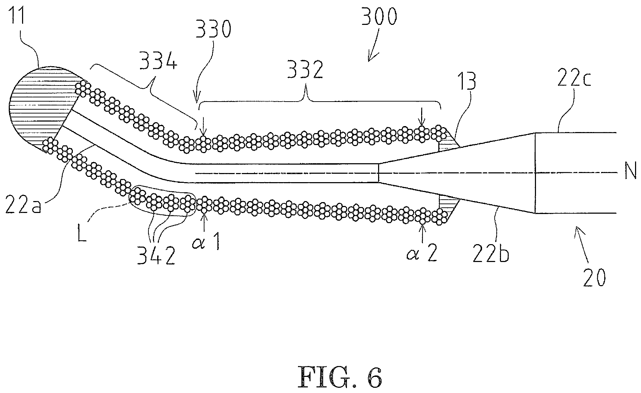

Next, the fourth embodiment of the guide wire of the present invention will be described with reference to FIG. 6. In FIG. 6, the left side corresponds to a distal side to be inserted in a body, while the right side corresponds to a proximal side to be operated by a technician such as a physician. Note that FIG. 6 schematically illustrates the guide wire, and the proportion in FIG. 6, including the configuration of the cross-section of the stranded wire, is different from actual one.

In a guide wire 300 of the fourth embodiment, a straight section 332 of a coil body 330 is tapered to a smaller diameter toward a curved section 334 (the distal side). That is, in the straight section 332, an outer diameter α1 at a portion on the curved section 334 side (the distal side) is smaller than an outer diameter α2 at a position on the rear side (the proximal side).

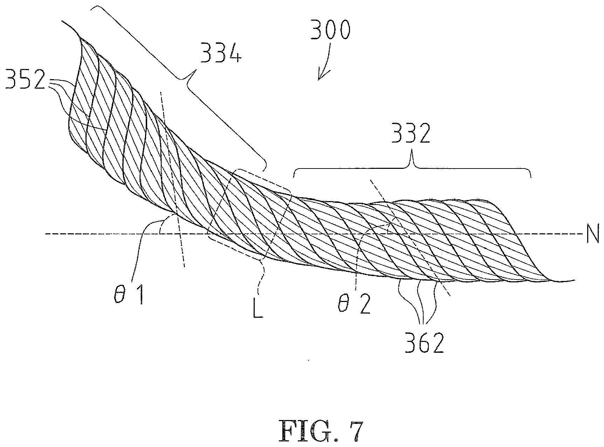

In this manner, the stranding angle or winding angle θ2 of the second stranded wires 362 forming the straight section 332 becomes gradually smaller toward the curved section 334 side (the distal side), and the second stranded wires 362 come to press the first stranded wires 352 forming the curved section 334, from the proximal side, as illustrated in FIG. 7.

That is, as illustrated in FIGS. 6 and 7, the stranded wires 352, 362 are in pressure contact with each other firmly at a border portion L between the curved section 334 and the straight section 332. As a result, it is possible to prevent more securely generation of a gap between turns of the stranded wires 342 (352, 362) even in a portion where a gap is generated easily when the distal portion of the guide wire 300 is curved (the border portion L between the straight section 332 and the curved section 334) .

Moreover, in the embodiment, the diameter is smallest at the proximal end part of the curved section 334, which improves flexibility of the curved section 334. Thus, even in a blood vessel curved three-dimensionally in a complicated manner, it is possible to sufficiently secure excellent blood vessel following property and the blood vessel selectivity of the distal portion of the guide wire 300.

In addition, in the straight section 332 of the guide wire 300 of the embodiment, the diameter becomes larger from the distal side to the proximal side. Thus, when the press-in operation is performed, sliding resistance to a blood vessel wall, for example, becomes larger toward the proximal side. Thus, if the distal portion (the curved section 334) of the guide wire 300 penetrates through a blood vessel wall and projects outside the blood vessel, and the operation is still continued thereafter, the sliding resistance of the straight section 332 to the blood vessel wall (a wall of the through-hole) becomes larger gradually, thus conveying such touch feeling to a hand of an operator.

Consequently, it becomes easy to stop the press-in operation in the guide wire 300 once and introduce again the distal portion of the guide wire 300 to a proper blood vessel. That is, the guide wire 300 of the fourth embodiment allows insertion of the distal portion to a target region safely and accurately. Furthermore, in such a case, with the use of a coil body whose outer diameter becomes smaller toward the distal side, the sliding resistance to a blood vessel wall, for example, in press-in operation further becomes larger toward the proximal side, thus easily conveying touch feeling in press-in operation in the guide wire to a hand of an operator.

## Claims

1. A guide wire (200; 300), comprising:
a core shaft (20); and
a coil body (230; 330) comprising a plurality of turns wound around a distal portion of the core shaft (20),
wherein the coil body (230, 330) is formed by winding at least one winding element into a helical coil structure,
wherein the coil body (230; 330) includes a straight section (232; 332) in a straight state from a proximal end of the coil body (230; 330) toward a distal direction and a curved section (234; 334) arranged at a distal side of the straight section (232; 332), and
wherein adjacent turns of the coil body (230; 330) are in contact with each other on an outer side of the curved section (234; 334)
**characterized in that**
each of the at least one winding element (242) is obtained by stranding a plurality of wires (242a), and
the coil body (230; 330) is formed by winding a plurality of winding elements (242; 342) into a helical coil structure.

2. A guide wire (100), comprising:
a core shaft (20); and
a coil body (130) comprising a plurality of turns wound around a distal portion of the core shaft (20),
wherein the coil body (130) is formed by winding at least one winding element into a helical coil structure, and
wherein the coil body (130) includes a straight section (132) in a straight state from a proximal end of the coil body (130) toward a distal direction and a curved section (134) arranged at a distal side of the straight section (132), and
wherein adjacent turns of the coil body (130) are in contact with each other on an outer side of the curved section (134)
**characterized in that**
the coil body (130) comprises a first coil portion (140) forming the curved section (134) and a second coil portion (150) forming the straight section (132),
the first coil portion (140) is formed by winding a plurality of winding elements (142) into a helical coil structure, each winding element (142) being obtained by stranding a plurality of wires (142a, 142b), and
the second coil portion (150) is formed of a single wire winding element wound into a helical coil structure.

3. The guide wire (100) according to claim 2,
wherein a proximal end of the first coil portion (140) and a distal end of the second coil portion (150) are joined to each other.

4. The guide wire (200; 300) according to claim 1,
wherein a winding angle (θ1) of first winding elements (252; 352) forming the curved section (234; 334) with respect to a longitudinal axis (N) of the straight section (232; 332) is larger than a winding angle (θ2) of second winding elements (262; 362) forming the straight section (232; 332) with respect to the longitudinal axis (N) of the straight section(232; 332).

5. The guide wire (200; 300) according to claim 4,
wherein the first winding elements (252; 352) forming the curved section (234; 334) are partially arranged substantially in parallel.

6. The guide wire (200; 300) according to claim 4 or 5,
wherein the first winding elements (252; 352) forming the curved section (234; 334) are in pressure contact with each other.

7. The guide wire (300) according to any one of claims 1 and 4 to 6,
wherein the straight section (332) is tapered to a smaller diameter toward the curved section (334).

## Patentansprüche

1. Führungsdraht (200; 300) mit:
einem Kernschaft (20); und
einem Spulenkörper (230; 330), der eine Vielzahl von um einen distalen Abschnitt des Kernschafts (20) gewundenen Windungen aufweist,
wobei der Spulenkörper (230, 330) aus wenigstens einem zu einer schraubenförmigen Spulenstruktur gewundenen Windungselement gebildet ist,
wobei der Spulenkörper (230; 330) einen geraden Abschnitt (232; 332) von einem proximalen Ende des Spulenkörpers (230; 330) aus in distaler Richtung und einen gekrümmten Abschnitt (234; 334) aufweist, der an einer distalen Seite des geraden Abschnitts (232; 332) angeordnet ist, und
wobei benachbarte Windungen des Spulenkörpers (230; 330) an der Außenseite des gekrümmten Abschnitts (234; 334) miteinander in Kontakt sind,
**dadurch gekennzeichnet, dass**
jedes des wenigstens einen Windungselements (242) aus einer Vielzahl von miteinander verdrillten Drähten (242a) gebildet ist, und
der Spulenkörper (230; 330) aus einer Vielzahl von zu einer schraubenförmigen Spulenstruktur gewundenen Windungselementen (242; 342) gebildet ist.

2. Führungsdraht (100) mit:
einem Kernschaft (20); und
einem Spulenkörper (130), der eine Vielzahl von um einen distalen Abschnitt des Kernschafts (20) gewundenen Windungen aufweist,
wobei der Spulenkörper (130) aus wenigstens einem zu einer schraubenförmigen Spulenstruktur gewundenen Windungselement gebildet ist,
wobei der Spulenkörper (130) einen geraden Abschnitt (132) von einem proximalen Ende des Spulenkörpers (130) aus in distaler Richtung und einen gekrümmten Abschnitt (134) aufweist, der einer distalen Seite des geraden Abschnitts (132) angeordnet ist, und
wobei benachbarte Windungen des Spulenkörpers (130) an der Außenseite des gekrümmten Abschnitts (134) miteinander in Kontakt sind,
**dadurch gekennzeichnet, dass**
der Spulenkörper (130) einen den gekrümmten Abschnitt (134) bildenden ersten Spulenabschnitt (140) und einen den geraden Abschnitt (132) bildenden zweiten Spulenabschnitt (150) aufweist,
der erste Spulenabschnitt (140) aus einer Vielzahl von zu einer schraubenförmigen Spulenstruktur gewundenen Windungselementen (142) gebildet ist, wobei jedes Windungselement (142) aus einer Vielzahl von miteiander verdrillten Drähten (142a, 142b) gebildet ist, und
der zweite Spulenabschnitt (150) aus einem einzigen zu einer schraubenförmigen Spulenstruktur gewundenen Windungselement gebildet ist.

3. Führungsdraht (100) nach Anspruch 2,
wobei ein proximales Ende des ersten Spulenabschnitts (140) und ein distales Ende des zweiten Spulenabschnitts (150) aneinandergefügt sind.

4. Führungsdraht (200; 300) nach Anspruch 1,
wobei ein Windungswinkel (θ1) der den gekrümmten Abschnitt (234; 334) bildenden ersten Windungselemente (252; 352) relativ zu einer Längsachse (N) des geraden Abschnitts (232; 332) größer ist als ein Windungswinkel (θ2) der den geraden Abschnitt (232; 332) bildenden zweiten Windungselemente (262; 362) relativ zur Längsachse (N) des geraden Abschnitt(232; 332).

5. Führungsdraht (200; 300) nach Anspruch 4,
wobei die den gekrümmten Abschnitt (234; 334) bildenden ersten Windungselemente (252; 352) teilweise im Wesentlichen parallel angeordnet sind.

6. Führungsdraht (200; 300) nach Anspruch 4 oder 5,
wobei die den gekrümmten Abschnitt (234; 334) bildenden ersten Windungselemente (252; 352) in Druckkontakt miteinander sind.

7. Führungsdraht (300) nach einem der Ansprüche 1 und 4 bis 6,
wobei sich der gerade Abschnitt (332) in Richtung des gekrümmten Abschnitts (334) im Durchmesser verjüngt.

## Revendications

1. Fil-guide (200 ; 300), comprenant :
une tige centrale (20) ; et
un corps de bobine (230 ; 330) comprenant une pluralité de spires enroulées autour d'une partie distale de la tige centrale (20),
dans lequel le corps de bobine (230, 330) est formé en enroulant au moins un élément d'enroulement en une structure de bobine hélicoïdale,
dans lequel le corps de bobine (230 ; 330) inclut une section droite (232 ; 332) dans un état droit depuis une extrémité proximale du corps de bobine (230 ; 330) vers une direction distale et une section incurvée (234 ; 334) agencée au niveau d'un côté distal de la section droite (232 ; 332), et
dans lequel des spires adjacentes du corps de bobine (230 ; 330) sont en contact mutuellement sur un côté extérieur de la section incurvée (234 ; 334)
**caractérisé en ce que**
chaque au moins un élément d'enroulement (242) est obtenu en toronnant une pluralité de fils (242a), et
le corps de bobine (230 ; 330) est formé en enroulant une pluralité d'éléments d'enroulement (242 ; 342) en une structure de bobine hélicoïdale.

2. Fil-guide (100), comprenant :
une tige centrale (20) ; et
un corps de bobine (130) comprenant une pluralité de spires enroulées autour d'une partie distale de la tige centrale (20),
dans lequel le corps de bobine (130) est formé en enroulant au moins un élément d'enroulement en une structure de bobine hélicoïdale, et
dans lequel le corps de bobine (130) inclut une section droite (132) dans un état droit depuis une extrémité proximale du corps de bobine (130) vers une direction distale et une section incurvée (134) agencée au niveau d'un côté distal de la section droite (132), et
dans lequel des spires adjacentes du corps de bobine (130) sont en contact mutuellement sur un côté extérieur de la section incurvée (134)
**caractérisé en ce que**
le corps de bobine (130) comprend une première partie de bobine (140) formant la section incurvée (134) et une seconde partie de bobine (150) formant la section droite (132),
la première partie de bobine (140) est formée en enroulant une pluralité d'éléments d'enroulement (142) en une structure de bobine hélicoïdale, chaque élément d'enroulement (142) étant obtenu en toronnant une pluralité de fils (142a, 142b), et
la seconde partie de bobine (150) est formée d'un élément d'enroulement à fil unique enroulé en une structure de bobine hélicoïdale.

3. Fil-guide (100) selon la revendication 2,
dans lequel une extrémité proximale de la première partie de bobine (140) et une extrémité distale de la seconde partie de bobine (150) sont jointes mutuellement.

4. Fil-guide (200 ; 300) selon la revendication 1,
dans lequel un angle d'enroulement (θ1) de premiers éléments d'enroulement (252 ; 352) formant la section incurvée (234 ; 334) par rapport à un axe longitudinal (N) de la section droite (232 ; 332) est supérieur à un angle d'enroulement (θ2) de seconds éléments d'enroulement (262 ; 362) formant la section droite (232 ; 332) par rapport à l'axe longitudinal (N) de la section droite (232 ; 332).

5. Fil-guide (200 ; 300) selon la revendication 4,
dans lequel les premiers éléments d'enroulement (252 ; 352) formant la section incurvée (234 ; 334) sont agencés partiellement sensiblement en parallèle.

6. Fil-guide (200 ; 300) selon la revendication 4 ou 5,
dans lequel les premiers éléments d'enroulement (252 ; 352) formant la section incurvée (234 ; 334) sont en contact de pression mutuellement.

7. Fil-guide (300) selon l'une quelconque des revendications 1 et 4 à 6,
dans lequel la section droite (332) se resserre jusqu'à un diamètre plus petit vers la section incurvée (334).
